# EUROPÄISCHE PATENTSCHRIFT

(11) **EP 0 707 458 B1**
(45) Veröffentlichungstag und Bekanntmachung des Hinweises auf die Patenterteilung: **10.09.1997**
(21) Anmeldenummer: 94920438.2
(22) Anmeldetag: 08.06.1994
(51) Int. Cl.: A61B 17/58, A61B 17/16

(54) **VORRICHTUNG ZUR OSTEOSYNTHESE VON KNOCHENFRAGMENTEN, INSBESONDERE ZUR FIXATION VON KNOCHENFRAKTUREN**
BONE FRAGMENT OSTEOSYNTHESIS DEVICE, IN PARTICULAR FOR FIXING BONE FRACTURES
DISPOSITIF D'OSTEOSYNTHESE DE FRAGMENTS OSSEUX, SERVANT NOTAMMENT A FIXER DES FRACTURES OSSEUSES

(30) Priorität: 06.07.1993 DE 4322507
(43) Veröffentlichungstag der Anmeldung: 24.04.1996
(60) Teilanmeldung: 96111282.8
(73) Patentinhaber: Gundolf, Ferdinand, A-6330 Kufstein (AT)
(72) Erfinder: Gundolf, Ferdinand, A-6330 Kufstein (AT)
(74) Vertreter: Popp, Eugen, Dr.
(86) Internationale Anmeldenummer: EP9401876
(87) Internationale Veröffentlichungsnummer: WO9501756

(56) Entgegenhaltungen:
- DE-A- 4 200 757

## Beschreibung

Die Erfindung betrifft eine Vorrichtung zur Osteosynthese von Knochenfragmenten, insbesondere zur Fixation von Knochenfrakturen gemäß dem Oberbegriff des Anspruches 1.

Als Hauptziel der Frakturbehandlung gilt die Wiederherstellung der Funktion der verletzten Extremität. Zur Vermeidung von Fehlstellungen und zur Verhütung von Frakturkrankheiten (Gelenkversteifungen und Weichteilschädigungen infolge zirkulatorischer Störungen) soll dem gebrochenen Knochen durch eine stabile Osteosynthese eine Festigkeit gegeben werden, die eine länger dauernde, äußere Fixation durch Gipsverbände oder dergleichen erübrigt und eine sofortige aktive Bewegungstherapie der verletzten Extremität erlaubt. Auch bei den wiederherstellenden Eingriffen am Skelett steht neben der zuverlässigen Verknöcherung die frühzeitige aktive Funktionsbehandlung im Vordergrund. Wichtig sind ferner die Abkürzung des Krankenhausaufenthaltes, die möglichst rasche Wiederherstellung der Tragfähigkeit des Knochens und vor allem auch die Abkürzung des operativen Eingriffs. Das vorgenannte Ziel wird in wesentlichen bereits erreicht durch die Vorrichtung gemäß der DE 42 00 757 A1, die ebenfalls auf den Erfinder der vorliegenden Erfindung zurückgeht und in der Praxis erfolgreich eingesetzt wird. Dabei hat sich jedoch gezeigt, daß es günstig wäre, das Spannband flexibler zu gestalten, um eine bessere Anpassung des Spannbandes an die Oberfläche des Knochens zu erreichen, derart, daß das Spannband über die gesamte Länge der Umschlingung vollflächig am Knochen anliegt.

Dieses Ziel wird erfindungsgemäß durch die kennzeichnenden Maßnahmen des Anspruches 1 erreicht. Dadurch, daß das Aufnahmeelement um eine sich etwa senkrecht zur Flachseite des Spannbandes erstreckende Achse schwenkbar gelagert ist, läßt sich das Spannband an konisch geformte Knochenabschnitte besser anpassen, derart, daß es längs der Umschlingung des Knochens weitgehend vollflächig an diesem anliegt.

Das Spannband umschlingt den Knochen bzw. die zu fixierende Knochenfraktur kragenartig. Auf diese Weise wird eine Linienberührung zwischen Spannband und Knochen weitgehend vermieden. Dementsprechend sanft und verträglich ist die Einwirkung der erfindungsgemäßen Vorrichtung auf dem Knochen.

Zur Erleichterung der Operation ist es vorteilhaft, an der dem Knochen zugewandten Seite des Endabschnitts des Spannbandes, an dem sich das Aufnahmeelement befindet, mindestens einen in den Knochen eindringenden Spike bzw. Dorn anzuordnen. Damit wird ein Verrutschen des Spannbandes beim Umschlingen des Knochens und Spannen vermieden Zu diesem Zweck können auch noch weitere Spikes bzw. Dorne an der dem Knochen zugewandten Seite des Spannbandes vorgesehen sein, jedoch vorzugsweise nur an der vorgenannten Aufnahmeelement zugeordneten Hälfte des Spannbandes.

Eine besonders einfache Konstruktion der erfindungsgemäßen Vorrichtung ergibt sich dadurch, daß das Aufnahmeelement durch einen zu einer Flachhülse gebogenen Spannbandabschnitt gebildet ist, dessen freier Querschnitt etwa dem Querschnitt des Spannbandes entspricht, derart, daß der andere Endabschnitt desselben mit Spielpassung durch die Flachhülse hindurchführbar ist.

Des weiteren ist in diesem Zusammenhang eine konstruktiv einfache Ausführungsform dadurch gekennzeichnet, daß die Schwenkachse des Aufnahmeelements an der dem Knochen zugewandten Seite des dem Aufnahmeelement zugeordneten Endabschnitts des Spannbandes vorsteht, wobei dieser vorstehende Abschnitt der Schwenkachse als Spike bzw. als Knochendorn ausgebildet ist. Insofern hat die Schwenkachse des Aufnahmeelements eine Doppelfunktion.

Um nach dem Spannen und Fixieren des Spannbandes eine erhöhte Rotationsstabilität zwischen Aufnahmeelement und dem zugeordneten Endabschnitt des Spannbandes zu erhalten, sind an der dem Spannband zugewandten Seite des schwenkbar gelagerten Aufnahmeelements und/oder an der dem Aufnahmeelement zugewandten Seite des Spannbandes sich sternartig um die Schwenkachse bzw. die die Schwenkachse aufnehmenden Bohrungen herum erstreckende Radialkerben ausgebildet.

Das Aufnahmeelement kann auch so ausgebildet sein, daß es sich in Richtung zum freien Endes des zugeordneten Spannband-Endabschnitts hin und/oder in entgegengesetzter Richtung trichterartig öffnet. Damit läßt sich eine erhöhte Anpaßbarkeit des Spannbandes an die Oberfläche des Knochens erhalten. Vorzugsweise ist auch bei dieser Ausführungsform das Aufnahmeelement nach Art einer Flachhülse ausgebildet.

Um die Flexibilität des Spannbandes zusätzlich zu erhöhen, können noch die Maßnahmen nach Anspruch 7 und/oder 8 vorgesehen sein. Dementsprechend soll das Aufnahmeelement innerhalb vorbestimmter Grenzen (etwa 5 bis 25°) relativ zur Schwenkachse kippbar sein, d.h. innerhalb vorgegebener Grenzen universal gelenkig gelagert sein. Vorzugsweise ist zu diesem Zweck die Schwenkachse flexibel ausgebildet oder das Aufnahmeelement mit etwas größerem Spiel an der Schwenkachse gehalten.

Zur Fixierung von losen Knochenfragmenten ist ein beweglicher Knochendorn gemäß den Ansprüchen 9 bis 11 hilfreich. Dieser verhindert auch ein unerwünschtes Verrutschen des Spannbandes beim Umschlingen des Knochens und Spannen.

Nachstehend werden Ausführungsbeispiele der erfindungsgemäßen Vorrichtung anhand der beigefügten schematischen Zeichnung näher erläutert. Es zeigen:
- Fig. 1: eine erfindungsgemäß ausgebildete Spannband-Vorrichtung in perspektivischer Ansicht;
- Fig. 2: die Spannband-Vorrichtung gemäß Fig. 1 unter Darstellung der Verschwenkbarkeit des Aufnahmeelements für den knochenentfernten Endabschnitt des Spannbandes;
- Fig. 3: eine Vorderansicht des knochennahen Endabschnitts des Spannbandes mit Spannband-Aufnahmeelement in Richtung des Pfeiles III in Fig. 1;
- Fig. 4: eine Unteransicht des Spannband-Aufnahmeelements unter Darstellung rotationsstabilisierender Radialkerben;
- Fig. 5: eine alternative Ausführungsform eines Spannband-Aufnahmeelements in Draufsicht;
- Fig. 6 und 7: bevorzugte Knochendorne in perspektivischer Ansicht; und
- Fig. 8: einen längs des Spannbandes verschiebbarer Knochendorn in Querschnittsansicht des Spannbandes.

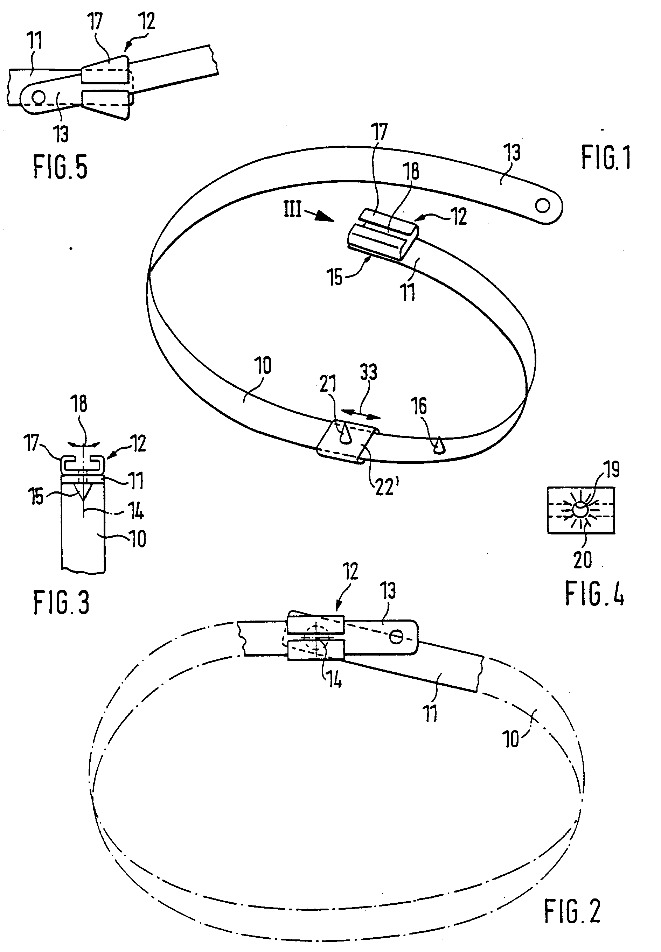

Die Vorrichtung zur Osteosynthese von Knochenfragmenten, insbesondere zur Fixation von Knochenfrakturen, gemäß den Figuren 1-4 umfaßt ein die Fraktur oder den Knochen im Bereich der zu behandelnden Stelle umschlingendes Spannmittel in Form eines flachbandartigen Spannbandes 10, an dessen einem, nämlich knochennahen Endabschnitt 11 ein Aufnahmeelement 12 angeordnet ist, durch das der andere, nämlich knochenentfernte Endabschnitt 13 des Spannbandes 10 hindurchführbar ist. Nach den Spannen des Spannbandes um den Knochen herum wird der durch das Aufnahmeelement 12 hindurchgeführte Teil des Endabschnitts 13 zur Festlegung der eingestellten Relativlage beider Endabschnitte 11 und 13 zueinander nach oben und zurückgebogen. Auf diese Weise ist der Endabschnitt 13 des Spannbandes 10 am Aufnahmeelement 12 fixiert. Diese Fixierung ist in den Figuren nicht näher dargestellt.

Wie die Figuren 2 und 3 erkennen lassen, ist das Aufnahmeelement 12 um eine sich senkrecht zur Flachseite des Spannbandes 10 erstreckende Achse 14 schwenkbar gelagert (siehe insbesondere Fig. 2). Dadurch ist eine bessere Anpassung des Spannbandes an die Oberfläche eines Knochens, insbesondere an eine etwa konisch geformte Knochenoberfläche möglich.

Zur Fixierung des knochennahen Endabschnitts 11 des Spannbandes 10 ist an der dem Knochen zugewandten Seite dieses Endabschnitts 11 mindestens ein in den Knochen eindringender Spike bzw. Dorn 15 angeordnet. Entsprechend Fig. 3 steht die Schwenkachse 14 des Aufnahmeelements 12 an der dem Knochen zugewandeten Seite des dem Aufnahmeelements 12 zugeordneten Endabschnitts 11 des Spannbandes 10 vor, wobei dieser vorstehende Abschnitt der Schwenkachse 14 als Spike bzw. Knochendorn 15 ausgebildet ist. Insofern besitzt die Schwenkachse 14 eine Doppelfunktion. Sie dient zugleich als Schwenkachse für das Aufnahmeelement 12 sowie zur Fixierung des knochennahen Endabschnitts 11 des Spannbandes 10 am Knochen. Damit wird ein Verrutschen des Spannbandes 10 beim Umschlingen des Knochens und Spannen verhindert.

Wie Fig. 1 erkennen läßt, ist an der dem Knochen zugewandten Seite des Spannbandes 10 ein weiterer Spike bzw. Knochendorn 16 ausgebildet. Dieser befindet sich in der dem Aufnahmeelement 12 zugeordneten Hälfte des Spannbandes 10.

Das Aufnahmeelement 12 ist bei der dargestellten Ausführungsform durch einen zu einer Flachhülse 17 gebogenen Spannbandabschnitt gebildet, dessen freier, etwa rechteckigförmiger Querschnitt dem Querschnitt des Spannbandes 10 entspricht, derart, daß der andere bzw. knochenentfernte Endabschnitt 13 des Spannbandes mit Spielpassung durch die Flachhülse 17 hindurchführbar ist. Diese Ausführungsform ist herstellungstechnisch besonders einfach. Vor allem läßt sich bei dieser Ausführungsform die Schwenkachse 14 einfach fixieren. Die Flachhülse 17 ist an der Oberseite durch einen Längsschlitz 18 geteilt. Zur Fixierung der Schwenkachse 14 sind die beiden oberen Hälften der Flachhülse 17 etwas auseinandergebogen. Nach der Fixierung der Schwenkachse 14 werden dann diese beiden Hälften wieder zurückgebogen, so daß die Flachhülse die in den Figuren 1 bis 3 dargestellte Gestalt aufweist. Die Schwenkachse 14 wird durch eine Niete gebildet, wobei der dem Knochen zugewandte Kopf eine spitzkegelige Form besitzt unter Ausbildung des oben erwähnten Spikes bzw. Knochendorns 15. Die Schwenkbarkeit des beschriebenen Aufnahmeelements 12 bzw. der Flachhülse 17 relativ zum Endabschnitt 11 des Spannbandes 10 ist in Fig. 3 durch den Doppelpfeil 18 angedeutet. Im übrigen wird diesbezüglich auf Fig. 2 verwiesen.

Entsprechend Fig. 4 sind an der dem Spannband 10 bzw. dem Endabschnitt 11 desselben zugewandten Seite des schwenkbar gelagerten Aufnahmeelements 12 sich sternartig um die Schwenkachse 14 bzw. die Aufnahmebohrung 19 für die Schwenkachse 14 herum erstreckende Radialkerben 20 ausgebildet. In gleicher Weise können auch an der dem Aufnahmeelement 12 zugewandten Seite des Spannbandes bzw. des Endabschnitts 11 desselben komplementäre Radialkerben vorgesehen sein. Auf diese Weise ist nach Fixierung des Spannbandes eine Rotationsstabilität zwischen Aufnahmeelement und dem zugeordneten Endabschnitt 11 des Spannbandes 10 gewährleistet.

Das Spannband sowie das Aufnahmeelement 12 sind vorzugsweise aus einer humanverträglichen Titanlegierung hergestellt.

In Fig. 5 ist eine Alternativlösung dargestellt, bei der das Aufnahmeelement 12 ebenfalls als Flachhülse 17 ausgebildet ist, die sich jedoch in Richtung zum freien Ende des zugeordneten Spannband-Endabschnitts 11 hin trichterartig öffnet. Dadurch wird eine erhöhte Flexibilität bzw. Anpaßbarkeit des Spannbandes an unterschiedliche Oberflächengestalten des zu behandelnden Knochens erhalten. Auch in entgegengesetzter Richtung kann die Flachhülse 17 trichterartig geöffnet sein. Auch ist ein Doppeltrichter nach beiden Seiten hin denkbar.

Um die Flexibilität der Vorrichtung zusätzlich zu erhöhen, kann das Aufnahmeelement innerhalb vorbestimmter Grenzen relativ zur Schwenkachse kippbar sein, d.h. innerhalb vorgegebener Grenzen universalgelenkig gelagert sein. Der Kippbereich beträgt etwa 5 bis 25° relativ zur Schwenkachse, und zwar allseitig. Konkret kann das vorgenannte Ziel dadurch erreicht werden, daß die Schwenkachse entweder flexibel ausgebildet, oder daß das Aufnahmeelement mit einem etwas größeren Spiel an der Schwenkachse gehalten ist.

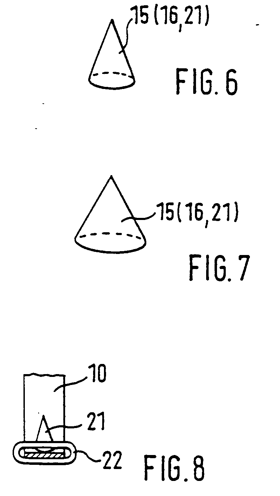

In den Figuren 6 und 7 sind zwei verschiedene Arten von Knochendornen dargestellt, wobei der Knochendorn gemäß Fig. 6 spitzkegelig und der Knochendorn gemäß Fig. 7 als flachgedrückter Spitzkegel ausgebildet ist. Die letztgenannte Ausführungsform zeichnet sich durch eine V-förmige Schneide aus, die ein Eindringen in den Knochen erleichtert.

In Fig. 1 ist noch ein weiterer Knochendorn 21 dargestellt, der auf einem U-förmigen Reiter 22' montiert längs des Spannbandes 10 verschiebbar ist. Diese Verschiebbarkeit ist in Fig. 1 mit dem Doppelpfeil 33 angedeutet. Um den Reiter 22' am Spannband 10 zu halten, sind die Ränder der beiden Schenkel unter Umgreifen der beiden Längsränder des Spannbandes 10 nach innen gebogen.

Statt des in Fig. 1 dargestellten U-förmigen Reiters 22' kann auch eine Flachhülse 22 vorgesehen sein, die längs des Spannbandes 10 verschiebbar ist. Es wird diesbezüglich auf Fig. 8 verwiesen. Der längs des Spannbandes 10 verschiebbare Knochendorn 21 ermöglicht eine individuelle Plazierung desselben. Insbesondere lassen sich mit diesem verschiebbaren Knochendorn lose Knochenfragmente fixieren. Auch läßt sich durch geeignete Plazierung des Knochendorns 21 vermeiden, daß das Spannband beim Umschlingen des Knochens und Spannen des Spannbandes in Knochenlängsrichtung verrutscht. Zu diesem Zweck wird der verschiebliche Knochendorn 21 vorzugsweise an der der Zugangsstelle durch die Fleischwunde hindurch gegenüberliegenden Seite des Knochens plaziert.

Die Reiterhülse 22 und der Reiter 22' sind vorzugsweise wieder aus einem Spannbandabschnitt hergestellt, auf jeden Fall aber aus dem gleichen Material wie das Spannband.

An durch die Wunde hindurch zugänglichen Stellen des Knochens empfiehlt es sich, diesen zur Plazierung eines Knochendorns 15, 16 oder 21 vorzubohren. Zu diesem Zweck kann ein Knochendorn-Bohrer dienen, der aus einem Schaft besteht, an dessen einem Ende ein Querriegel und an dessen anderem Ende ein spitzkegeliger Bohrdorn angeordnet ist. Der Bohrdorn ist in der Art eines Holzbohrers ausgebildet. Der Querriegel dient als Griff zur Übertragung eines Bohrdrehmoments.

## Patentansprüche

1. Vorrichtung zur Osteosynthese von Knochenfragmenten, insbesondere zur Fixation von Knochenfrakturen, mit einem die Fraktur oder den Knochen im Bereich der zu behandelnden Stelle umschlingenden Spannmittel, wobei das Spannmittel ein nach Art einer Rohr- oder Schlauchschelle ausgebildetes flachbandartiges Spannband (10) ist, an dessen einem, nämlich knochennahen Endabschnitt (11) ein Aufnahmeelement (12) angeordnet ist, durch das der andere, nämlich knochenentfernte Endabschnitt (13) des Spannbandes (10) hindurchführbar ist, um dann zur Festlegung der Umschlingung unter Spannung am Aufnahmeelement (12) fixiert werden zu können,
dadurch **gekennzeichnet,** daß
das Aufnahmeelement (12) um eine sich etwa senkrecht zur Flachseite des Spannbandes (10) erstreckende Achse (14) schwenkbar gelagert ist.

2. Vorrichtung nach Anspruch 1,
dadurch **gekennzeichnet,** daß
an der dem Knochen zugewandten Seite des knochennahen Endabschnitts (11) des Spannbandes (10) mindestens ein in den Knochen eindringender Spike bzw. Dorn (15) angeordnet ist.

3. Vorrichtung nach Anspruch 1 oder 2,
daduch **gekennzeichnet,** daß
das Aufnahmeelement (12) durch einen zu einer Flachhülse (17) gebogenen Spannbandabschnitt gebildet ist, dessen freier, etwa rechteckförmiger Querschnitt dem Querschnitt des Spannbandes (10) entspricht, derart, daß der andere bzw. knochenentfernte Endabschnitt (13) desselben mit Spielpassung durch die Flachhülse (17) hindurchführbar ist.

4. Vorrichtung nach einem der Ansprüche 1 bis 3,
dadurch **gekennzeichnet,** daß
die Schwenkachse (14) des Aufnahmeelements (12) an der dem Knochen zugewandten Seite des dem Aufnahmeelement (12) zugeordneten Endabschnitts (11) des Spannbandes (10) vorsteht, wobei dieser vorstehende Abschnitt der Schwenkachse (14) als Spike bzw. Dorn (15) ausgebildet ist.

5. Vorrichtung nach einem der Ansprüche 1 bis 4,
dadurch **gekennzeichnet,** daß
an der dem Spannband (10) zugewandten Seite des schwenkbar gelagerten Aufnahmeelements (12) und/oder an der dem Aufnahmeelement (12) zugewandten Seite des Spannbandes (10) sich sternartig um die Schwenkachse (14) bzw. deren Aufnahmebohrungen (19) herum erstreckende Radialkerben (20) ausgebildet sind.

6. Vorrichtung nach dem Oberbegriff des Anspruches 1,
dadurch **gekennzeichnet,** daß
das Aufnahmeelement (12) sich in Richtung zum freien Ende des zugeordneten Spannband-Endabschnitts (11) hin und/oder in entgegengesetzter Richtung trichterartig öffnet.

7. Vorrichtung nach einem der Ansprüche 1 bis 5,
dadurch **gekennzeichnet,** daß
das Aufnahmeelement (12) innerhalb vorbestimmter Grenzen, etwa 5 bis 25°, relativ zur Schwenkachse (14) allseitig kippbar ist, d.h. innerhalb vorgegebener Grenzen universal gelenkig gelagert ist.

8. Vorrichtung nach Anspruch 7,
dadurch **gekennzeichnet,** daß
die Schwenkachse (14) des Aufnahmeelements (12) flexibel ausgebildet ist und/oder das Aufnahmeelement (12) mit etwas größerem Spiel an der Schwenkachse (14) gehalten ist.

9. Vorrichtung, insbesondere nach einem oder mehreren der Ansprüche 1 bis 8,
dadurch **gekennzeichnet,** daß
mindestens ein längs des Spannbandes (10) versetzbarer Knochenspike bzw. -dorn (21) vorgesehen ist.

10. Vorrichtung nach Anspruch 9,
dadurch **gekennzeichnet,** daß
der versetzbare Knochendorn (21) an der dem Knochen zugewandten Seite einer längs des Spannbandes (10) verschiebbaren Reiter-Hülse, insbesondere Flachhülse (22), angeordnet ist.

11. Vorrichtung nach Anspruch 10,
dadurch **gekennzeichnet,** daß
der versetzbare Knochendorn (21) an der dem Knochen zugewandten Seite eines längs des Spannbandes (10) verschiebbaren, etwa U-förmigen Reiters (22') angeordnet ist.

## Claims

1. Device for osteosynthesis of bone fragments, in particular for fixation of bone fractures, with a tightening means which loops round the fracture or the bone in the region of the site which is to be treated, the tightening means being a flat belt-like tightening strap (10) which is designed in the manner of a tube clip or hose clip and on one end section (11) of which, namely the end section proximal to the bone, there is arranged a receiving element (12) through which the other end section (13) of the tightening strap (10), namely the end section remote from the bone, can be guided in order then to be able to be fixed so as to secure the loop by tightening on the receiving element (12), characterized in that the receiving element (12) is mounted pivotably about an axle (14) extending approximately perpendicular to the flat side of the tightening strap (10).

2. Device according to Claim 1, characterized in that the end section (11) of the tightening strap (10) proximal to the bone has, on its side facing the bone, at least one spike (15) penetrating into the bone.

3. Device according to Claim 1 or 2, characterized in that the receiving element (12) is formed by a section of the tightening strap which has been bent to give a flat sleeve (17) and whose free and approximately rectangular cross-section corresponds to the cross-section of the tightening strap (10) such that the other end section (13) thereof, remote from the bone, can be guided through the flat sleeve (17) with a clearance fit.

4. Device according to one of Claims 1 to 3, characterized in that the pivot axle (14) of the receiving element (12) projects on the side, facing the bone, of the end section (11) of the tightening strap (10) allocated to the receiving element (12), this projecting section of the pivot axle (14) being designed as a spike (15).

5. Device according to one of Claims 1 to 4, characterized in that radial notches (20) extending in a star shape about the pivot axle (14) and its receiving bores (19) are designed on that side of the pivotably mounted receiving element (12) facing the tightening strap (10) and/or on that side of the tightening strap (10) facing the receiving element (12).

6. Device according to the preamble of Claim 1, characterized in that the receiving element (12) opens in a funnel shape in the direction towards the free end of the associated end section (11) of the tightening strap and/or in the opposite direction.

7. Device according to one of Claims 1 to 5, characterized in that the receiving element (12) can be tilted to all sides relative to the pivot axle (14) within predetermined limits, approximately 5 to 25°, i.e. is mounted as universal hinge within predetermined limits.

8. Device according to Claim 7, characterized in that the pivot axle (14) of the receiving element (12) is designed to be flexible and/or the receiving element (12) is held on the pivot axle (14) with somewhat greater play.

9. Device, in particular according to one or more of Claims 1 to 8, characterized in that at least one bone spike (21) is provided which can be moved along the tightening strap (10).

10. Device according to Claim 9, characterized in that the movable bone spike (21) is arranged on the side, facing the bone, of a slide sleeve, in particular flat sleeve (22), which can be displaced along the tightening strap (10).

11. Device according to Claim 10, characterized in that the movable bone spike (21) is arranged on the side, facing the bone, of an approximately U-shaped slide (22') which can be displaced along the tightening strap (10).

## Revendications

1. Dispositif d'ostéosynthèse de fragments osseux, servant notamment à fixer des fractures osseuses, avec un moyen de mise sous tension entourant la fracture ou l'os dans la zone de l'endroit à traiter, dans lequel le moyen de mise sous tension est une bande plate de mise sous tension (10) conçue sous la forme d'une bride de type tubaire ou tuyau, en l'une des extrémités de laquelle, notamment en l'extrémité (11) proche de l'os, est agencé un élément récepteur (12) au travers duquel l'autre extrémité de la bande (10), notamment l'extrémité éloignée (13) de l'os, peut être engagée pour pouvoir être fixée à cet élément récepteur (12) pour tenir la boucle sous tension, caractérisé en ce que l'élément récepteur (12) est monté de telle sorte à être articulé autour d'un axe (14) sensiblement perpendiculaire au côté plat de la bande (10) de mise sous tension.

2. Dispositif selon la revendication 1, caractérisé en ce qu'au moins une pointe ou un ardillon (15) pénétrant dans l'os est agencé en la face orientée vers l'os de l'extrémité (11) proche de l'os de la bande plate de mise sous tension (10).

3. Dispositif selon la revendication 1 ou 2, caractérisé en ce que l'élément récepteur (12) est réalisé par une partie de la bande de mise sous tension pliée sous la forme d'une douille plate (17) dont la section libre sensiblement rectangulaire correspond à celle de la bande de mise sous tension (10) de telle sorte que l'autre extrémité (13) éloignée de l'os peut être engagée avec un jeu au travers de la douille plate (17).

4. Dispositif selon l'une des revendications 1 à 3, caractérisé en ce que l'axe d'articulation (14) de l'élément récepteur (12) protubère de la face orientée vers l'os de l'extrémité (11) de la bande de mise sous tension (10) correspondante à l'élément récepteur (12), laquelle portion protubérante de l'axe d'articulation (14) prend la forme d'une pointe ou d'un ardillon (15).

5. Dispositif selon l'une des revendications 1 à 4, caractérisé en ce que des nervures radiales (20) sont réalisées en la face de la bande de mise sous tension (10) orientée vers l'élément récepteur articulé (12) et/ou en la face de l'élément récepteur (12) orientée vers la bande de mise sous tension (10), et se prolongent à la manière d'une étoile autour de l'axe d'articulation (14) ou de son orifice de réception (19).

6. Dispositif selon la revendication 1, caractérisé en ce que l'élément récepteur (12) s'ouvre, en direction de son extrémité ouverte pour l'extrémité correspondante (11) de la bande de mise sous tension, en forme d'entonnoir dans le sens et/ou dans le sens contraire.

7. Dispositif selon l'une des revendications 1 à 5, caractérisé en ce que l'élément récepteur (12) est monté en basculement de tout côté par rapport à l'axe d'articulation (14) à l'intérieur de limites prédéterminées de l'ordre de 5 à 25°, c'est-à-dire est monté selon une articulation universelle à l'intérieur de limites prédéterminées.

8. Dispositif selon la revendication 7, caractérisé en ce que l'axe d'articulation (14) de l'élément récepteur (12) est flexible et/ou en ce que l'élément récepteur (12) est retenu à l'axe d'articulation (14) avec un jeu sensiblement plus important.

9. Dispositif selon l'une ou plusieurs des revendications 1 à 8, caractérisé en ce qu'il est prévu au moins une pointe ou un ardillon vers l'os (21) réglable le long de la longueur de la bande de mise sous tension (10).

10. Dispositif selon la revendication 9, caractérisé en ce que la pointe vers l'os réglable (21) est agencée en la face orientée vers l'os sur une douille, notamment une douille plate (22), montée coulissante le long de la bande de mise sous tension (10).

11. Dispositif selon la revendication 9, caractérisé en ce que la pointe vers l'os réglable (21) est agencée en la face orientée vers l'os sur une douille sensiblement en forme de U (22') coulissante le long de la bande de mise sous tension (10).
